# EUROPEAN PATENT SPECIFICATION

(11) **EP 2 484 757 B1**
(45) Date of publication and mention of the grant of the patent: **29.04.2015**
(21) Application number: 10820641.8
(22) Date of filing: 30.09.2010
(51) Int. Cl.: C12N 7/00, A61K 39/145, A61K 39/165, A61K 39/17, A61K 39/20, A61K 39/21, A61K 39/255, A61K 39/285, A61P 31/12, A61P 31/16, C12N 7/04, C12N 15/09, C12N 9/10

(54) **METHOD FOR THE PREPARATION OF VIRUS EXPRESSING ALPHA-GALACTOSYL EPITOPES**
VERFAHREN ZUR HERSTELLUNG EINES VIRUSES, WELCHES ALPHA-GALAKTOSYL-EPITOPE EXPRIMIERT
PROCEDE DE PREPARATION DE VIRUS EXPRIMANT DES EPITOPES D'ALPHA-GALACTOSYLE

(30) Priority: 30.09.2009 JP 2009227753
(43) Date of publication of application: 08.08.2012
(73) Proprietor: National University Corporation Obihiro University of Agriculture and Veterinary Medicine, Obihiro-shi, Hokkaido 080-8555 (JP)
(72) Inventor: OGAWA, Haruko, Inada-cho, Obihiro-shi Hokkaido 080-8555 (JP); IMAI, Kunitoshi, Inada-cho, Obihiro-shi Hokkaido 080-8555 (JP); TAGAMI, Takahiro, Tsukuba-shi Ibaraki 305-0901 (JP)
(74) Representative: Vossius & Partner Patentanwälte Rechtsanwälte mbB
(86) International application number: PCT/JP2010/067082
(87) International publication number: WO 2011/040526

(56) References cited:
- WO-A2-2008/118487
- US-A1- 2009 123 494
- US-A1- 2009 123 494
- TAKEUCHI Y ET AL: "SENSITIZATION OF CELLS AND RETROVIRUSES TO HUMAN SERUM BY (ALPHA1-3) GALACTOSYLTRANSFERASE", NATURE: INTERNATIONAL WEEKLY JOURNAL OF SCIENCE, NATURE PUBLISHING GROUP, UNITED KINGDOM, vol. 379, no. 6560, 4 January 1996 (1996-01-04), pages 85-88, XP000569530, ISSN: 0028-0836, DOI: 10.1038/379085A0
- HITOSHI N ET AL: "Efficient selection for high-expression transfectants with a novel eukaryotic vector", GENE, ELSEVIER, AMSTERDAM, NL, vol. 108, no. 2, 15 December 1991 (1991-12-15), pages 193-199, XP023541480, ISSN: 0378-1119, DOI: 10.1016/0378-1119(91)90434-D [retrieved on 1991-12-15]
- IKEMATSU SHINYA ET AL: "Transgenic mouse lines with ectopic expression of alpha-1,3-galactosyltransferase: Production and characteristics", GLYCOBIOLOGY, OXFORD UNIVERSITY PRESS, US, vol. 3, no. 6, 1 January 1993 (1993-01-01) , pages 575-580, XP002697680, ISSN: 0959-6658
- ABDEL-MOTAL USSAMA ET AL: "Increased immunogenicity of human immunodeficiency virus gp120 engineered to express Gal alpha 1-3Ga1 beta 1-4GlcNAc-R epitopes", JOURNAL OF VIROLOGY, THE AMERICAN SOCIETY FOR MICROBIOLOGY, US, vol. 80, no. 14, 1 July 2006 (2006-07-01), pages 6943-6951, XP002543575, ISSN: 0022-538X, DOI: 10.1128/JVI.00310-06
- ABDEL-MOTAL USSAMA M ET AL: "Immunogenicity of influenza virus vaccine is increased by anti-Gal-mediated targeting to airitigen-presenting cells", JOURNAL OF VIROLOGY, THE AMERICAN SOCIETY FOR MICROBIOLOGY, US, vol. 81, no. 17, 1 September 2007 (2007-09-01), pages 9131-9141, XP002543576, ISSN: 0022-538X, DOI: 10.1128/JVI.00647-07
- PREECE, AF. ET AL.: 'Expression of ABO or related antigenic carbohydrates on viral envelopes leads to neutralization in the presence of serum containing specific natural antibodies and complement.' BLOOD vol. 99, 2002, pages 2477 - 2482, XP008155431
- ABDEL-MOTAL, UM. ET AL.: 'Immunogenicity of influenza virus vaccine is increased by anti-gal-mediated targeting to antigen- presenting cells.' J VIROL. vol. 81, 2007, pages 9131 - 9141, XP008155433

### SEQUENCE LISTING

<110> National University Corporation Obihiro University of Agriculture and Veterinary Medicine
<120> Preparation method of virus expressing alpha-galactose epitope and vaccine
<130> 105103H
<160> 3
<170> PatentIn version 3.5
<210> 1
   <211> 3450
   <212> DNA
   <213> Mouse
<400> 1
   cgtcttagga ggctggagat tctgggtgga gccctagccc tgccttttct tagctggctg     60
   acaccttccc ttgtagactc ttcttggaat gagaagtacc gattctgctg aagacctcgc    120
   gctctcaggc tctgggagtt ggaaccctgt accttccttt cctctgctga gccctgcctc    180
   cttcggcagg ccagagctcg acagaagctc ggttgctttg ctgtttgctt tggagggaac    240
   acagctgacg atgaggctga ctttgaactc aagagatctg cttaccccag tctcctggaa    300
   ttaaaggcct gtactacctt gcctggacct aagattttca tgatcactat gcttcaagat    360
   ctccatgtca acaagatctc catgtcaaga tccaagtcag aaacaagtct tccatcctca    420
   agatctggat cacaggagaa aataatgaat gtcaagggaa aagtaatcct gttgatgctg    480
   attgtctcaa ccgtggttgt cgtgttttgg gaatatgtca acagcccaga cggctctttc    540
   ttgtggatat atcacacaaa aattccagag gttggtgaga acagatggca gaaggactgg    600
   tggttcccaa gctggtttaa aaatgggacc cacagttatc aagaagacaa cgtagaagga    660
   cggagagaaa agggtagaaa tggagatcgc attgaagagc ctcagctatg ggactggttc    720
   aatccaaaga accgcccgga tgttttgaca gtgaccccgt ggaaggcgcc gattgtgtgg    780
   gaaggcactt atgacacagc tctgctggaa aagtactacg ccacacagaa actcactgtg    840
   gggctgacag tgtttgctgt gggaaagtac attgagcatt acttagaaga ctttctggag    900
   tctgctgaca tgtacttcat ggttggccat cgggtcatat tttacgtcat gatagatgac    960
   acctcccgga tgcctgtcgt gcacctgaac cctctacatt ccttacaagt ctttgagatc   1020
   aggtctgaga agaggtggca ggatatcagc atgatgcgca tgaagaccat tggggagcac   1080
   atcctggccc acatccagca cgaggtcgac ttcctcttct gcatggacgt ggatcaagtc   1140
   tttcaagaca acttcggggt ggaaactctg ggccagctgg tagcacagct ccaggcctgg   1200
   tggtacaagg ccagtcccga gaagttcacc tatgagaggc gggaactgtc ggccgcgtac   1260
   attccattcg gagaggggga tttttactac cacgcggcca tttttggagg aacgcctact   1320
   cacattctca acctcaccag ggagtgcttt aaggggatcc tccaggacaa gaaacatgac   1380
   atagaagccc agtggcatga tgagagccac ctcaacaaat acttcctttt caacaaaccc 1440
   actaaaatcc tatctccaga gtattgctgg gactatcaga taggcctgcc ttcagatatt 1500
   aaaagtgtca aggtagcttg gcagacaaaa gagtataatt tggttagaaa taatgtctga 1560
   cttcaaattg tgatggaaac ttgacactat tactctggct aattcctcaa acaagtagca 1620
   acacttgatt tcaactttta aaagaaacaa tcaaaaccaa aacccactac catggcaaac 1680
   agatgatttc tcctgacacc ttgagcctgt aatatgtgag aaagagtcta tggcaagtaa 1740
   tcaggtataa attctcaatg atttcttata tattctgggt cttgggaaaa cttgattcta 1800
   gaaatcaaaa ttaatttgac aaaggaaaag cagatgccgg aaacttcttc ccagtctgtc 1860
   atacaattca ccactggcca ggtgctgaga gaagcattag ggaacagtgt gggttgtgtc 1920
   agagttggac ggctccatcc ctttggcttc attatcttcc tcctcatgga gattctaaag 1980
   caacccagag aggctttgca gccagagacc tttaataagg atgccaatgt gaccatcagt 2040
   ctgtaaaagc tgatggctcc aggagcgctg gcagtccagg ccccactagg ctattgtttc 2100
   tgtcctgggc ataaaggagg cagagagtgc caataggtac tttggtggca catgttcaga 2160
   gtccaggaaa aatcaagggt gaccacttag agggacatag gacttggggt tggtgattga 2220
   actgagttac aaacacagac agctttcttc aggatgacta acagcaggaa ttgaatggaa 2280
   agtgtgttca ttttgttttg cccaaattgt attcatgctg ttagctttgt gtgttgagcc 2340
   ctgtggagag ggtgtgactg tatcagggaa ggagagtacc tcagcggact gaggaccagc 2400
   accctattat atcagaagac aatctctcat catcaggtcc tacctacaac ctgctctgaa 2460
   cctccgagtt cctcagccca tcgtgttcca gtgtgggggc ctgtatggag caggtgactg 2520
   aagacaaagc cccctgtcac atgacctcat ttcccctgct ctagtactat gcaagtgtga 2580
   cagccagcca gccagatgta ctggacaaca taggaaccga ctttatggca atgggagccg 2640
   cagtcactac aacggagctg ctgaaggttc tgttccccgc tctgagagcc tgcaggagcc 2700
   cctgtatagg tggttctcaa cctatgggtc gcgacccctt tgggaagtgt taaatgaccc 2760
   tttcacaggt gtcccctaag acggttaaaa aacatagata tttccactct gactggtaac 2820
   agtagcagaa ttacagttat gaaatagcaa gggaaataat tctggggttc gtgtcatcca 2880
   taccatgagg agctacatta ggtcacatca ttagggaagt tgagaagcat agctctactt 2940
   gggtatttaa gcaaattatg caaagggggt tgtcgctctg tgttctgtgt atgcatatat 3000
   ttatattttg cttgtcttcc agtttaggtc aatctgtttc ttcctttaag cagtttattt 3060
   aaaaggccat tgcaccatct tggtgaacag catgaggggt ttcaataaaa aataggatct 3120
   tacctttgtc cacagggctc tacctcttac ttttcaattg tgaacaaaaa aggtcgcaca 3180
   cccagaggca acaaaaccca cagaattcct gaaccaatgg gagatgccaa tggaagcaga 3240
   gcttgcacat ctgctaaaaa ttctgcctct ctgtcactgt gctggatccg tctaaagtgg 3300
   gacagttcaa tggtctgaaa gtttcaaaaa ggctggggaa tttgagggga ttttttttta 3360
   aaataaaatt gatccaagtt taaatctcta atgagtaagc ttaggatttt attaaaggta 3420
   atttttagac attcttcaaa ataagaattc 3450
<210> 2
   <211> 1343
   <212> DNA
   <213> Pig
<400> 2
   cgggggccat ccccgagcgc acccagcttc tgccgatcag gagaaaataa tgaatgtcaa 60
   aggaagagtg gttctgtcaa tgctgcttgt ctcaactgta atggttgtgt tttgggaata 120
   catcaacaga aacccagaag ttggcagcag tgctcagagg ggctggtggt ttccgagctg 180
   gtttaacaat gggactcaca gttaccacga agaagaagac gctataggca acgaaaagga 240
   acaaagaaaa gaagacaaca gaggagagct tccgctagtg gactggttta atcctgagaa 300
   acgcccagag gtcgtgacca taaccagatg gaaggctcca gtggtatggg aaggcactta 360
   caacagagcc gtcttagata attattatgc caaacagaaa attaccgtgg gcttgacggt 420
   ttttgctgtc ggaagataca ttgagcatta cttggaggag ttcttaatat ctgcaaatac 480
   atacttcatg gttggccaca aagtcatctt ttacatcatg gtggatgata tctccaggat 540
   gcctttgata gagctgggtc ctctgcgttc ctttaaagtg tttgagatca agtccgagaa 600
   gaggtggcaa gacatcagca tgatgcgcat gaagaccatc ggggagcaca tcctggccca 660
   catccagcac gaggtggact tcctcttctg cattgacgtg gatcaggtct tccaaaacaa 720
   ctttggggtg gagaccctgg gccagtcggt ggctcagcta caggcctggt ggtacaaggc 780
   acatcctgac gagttcacct acgagaggcg gaaggagtcc gcagcctaca ttccgtttgg 840
   ccagggggat ttttattacc acgcagccat tttgggggga acacccactc aggttctaaa 900
   catcactcag gagtgcttca agggaatcct ccaggacaag gaaaatgaca tagaagccga 960
   gtggcatgat gaaagccatc taaacaagta tttccttctc aacaaaccca ctaaaatctt 1020
   atccccagaa tactgctggg attatcatat aggcatgtct gtggatatta ggattgtcaa 1080
   gatagcttgg cagaaaaaag agtataattt ggttagaaat aacatctgac tttaaattgt 1140
   gccagcagtt ttctgaattt gaaagagtat tactctggct acttcctcag agaagtagca 1200
   cttaatttta acttttaaaa aatactaaca aaataccaac acagtaagta catattattc 1260
   ttccttgcaa ctttgagcct tgtcaaatgg gagaatgact ctgtagtaat cagatgtaaa 1320
   ttcccaatga tttcttatct gcg 1343
<210> 3
   <211> 1828
   <212> DNA
   <213> Cattle
<400> 3
   ccgggggccg ggccgagctg ggagcgtcga gcccgctgcc cagcgcccgc cggctccctc      60
   gcgcccctgc ccgccgcccc ggaggagcgc ccggcggccg gccgacggga gcgcagcggc     120
   acaccccgcc ccggcacgcc cgcggggctc gggaggaggc agcgcgccga ctgttccggc     180
   agccgaggac gccgccgggg agccgaggcg ccggccagcc cccagcgcgc ccagcttctg     240
   cggatcaggg aaaccacgtg tcctcaagtg gccagccagc tgtccccaag aggaacttgc     300
   ctggcatttg cacggaaaga cgagacactt cacaaaatca acggagtcag aaggctgcac     360
   cttcgcttcc tcccagccct gcctccttct gcagaacgga gctcagtaga acttggtact     420
   tttgcctttt actctaggag gagagaagca gacgatgagg agaaaataat gaatgtcaaa     480
   ggaaaagtga ttctgtcaat gctggttgtc tcaactgtca ttgttgtgtt ttgggaatat     540
   atccacagcc cagaaggctc tttgttctgg ataaacccat caagaaaccc agaagttggt     600
   ggcagcagca ttcagaaggg ctggtggctt ccgagatggt ttaacaatgg ttaccatgaa     660
   gaagatggag acataaacga agaaaaggaa caaagaaacg aagacgaaag caagcttaag     720
   ctatcggact ggttcaaccc atttaaacgc cccgaggttg tgaccatgac gaagtggaag     780
   gctccagtgg tgtgggaagg cacttacaac agagccgtct tagacaatta ttatgccaag     840
   cagaaaatta ccgtcggcct gacggttttc gccgtcggaa gatacattga gcattacttg     900
   gaggagttct taacgtctgc taataagcac ttcatggtgg gccacccagt catcttttat     960
   atcatggtag atgatgtctc caggatgcct ttgatagagt tgggtcctct gcgctccttc    1020
   aaagtgttta agatcaagcc tgagaagagg tggcaggaca tcagcatgat gcgcatgaag    1080
   actatcgggg agcacattgt ggcccacatc cagcatgagg ttgacttcct tttctgcatg    1140
   gatgtggacc aggtcttcca agacaagttt ggggtggaga ccctgggcga gtcggtggcc    1200
   cagctacaag cctggtggta caaggcagat cccaatgact tcacctacga gaggcggaag    1260
   gagtctgcag catacattcc cttcggcgaa ggggattttt attaccatgc agccattttt    1320
   gggggaacac ccactcaggt ccttaacatc acccaggaat gcttcaaagg aatcctcaag    1380
   gacaagaaaa atgacataga agcccaatgg catgatgaaa gccatctaaa caagtatttc    1440
   cttctcaaca aacctactaa aatcttatcc ccggaatact gctgggatta tcacataggc    1500
   ctacctgcgg atattaagct tgtcaagatg tcttggcaga caaaagagta taatgtggtt    1560
   agaaataatg tctgactttg tgccagtaca tttctgaatt tgagagagta ttattctggc    1620
   tacttcctca gaaaagtaac acttaatttt aacttaaaaa aaaatactaa caaaagacca    1680
   acacagcaaa tacatattat ttctccttgt aactttgagc cttgtaatac gggagaatga    1740
   acctgtggta atcagatgta aattcccagt gatttcttac ctatttttgg ttgtgggggc    1800
   ggggaatgga tacaccatca gttgaacc                                        1828

## Description

This application claims priority from Japanese Patent Application 2009-227753, filed September 30, 2009.

The present disclosure relates to a method for producing a virus expressing α-Gal by inoculating a virus into a cell line that has been made to express α-galactose epitope (Galα1-3Galβ1 -4GlcNAc-R, sometimes abbreviated to α-Gal hereinafter) by the introduction of a gene, and to a method for producing a vaccine using this virus.

As the worldwide spread of highly pathogenic avian influenza and swine-origin pandemic influenza has continued, the danger of widespread infection not just of domestic fowl, but also of humans has been pointed out. Thus, there is a greater need than ever to tackle the prevention and control of influenza. Vaccines prepared in developing eggs are considered effective for preventing influenza. Although immunopotentiating agents presenting the risk of side effects are currently not contained in human influenza vaccines, when a new influenza develops in the future, it is conceivable that a vaccine might be employed in an urgent fashion. Thus, the development of safe methods for intensifying the effects of vaccines is an important topic. Further, in order to inoculate vaccines to more people, it is necessary to ensure the technical means for supply of vaccines that are effective even in small amounts.

A method of preparing a vaccine for the influenza virus using a cell culture system is described in Patent Reference 1, for example. This method employs avian embryonic stem cells. However, there is no disclosure of a method of safely enhancing the effect of the vaccine.

α-Galactose epitope (α-Gal) is a glycoantigen with a structure that is similar to that of ABO blood type antigens. It is acted upon by α1,3-galactosyltransferase (sometimes abbreviated to α1,3GT hereinafter) and expressed on the cell surface. α-Gal is expressed in most mammals. However, the α1,3GT gene does not function in humans and Aves, in which there is no expression of α-Gal. Thus, α-Gal is recognized as a foreign antigen in humans and Aves, which have natural antibodies to α-Gal (anti-α-Gal antibodies) (Nonpatent Reference 1). The reaction of α-Gal and anti-α-Gal antibody is the cause of hyperacute rejection reactions in intra-species organ transplants and the like between swine and human, for example. One of the present inventors, Ogawa, has investigated techniques of controlling α-Gal and anti-α-Gal antibodies in xenogeneic transplantation. She has participated in research into the antigenic potentiation of cancer vaccines utilizing this reaction, and confirmed its efficacy (Nonpatent Reference 2).

If the above method of antigenic potentiation employing α-Gal could be applied to influenza, and an influenza virus made to express α-Gal could be prepared, anti-α-Gal antibodies might bind to the virus in humans and Aves, and then bind to the Fcγ receptor of antigen-presenting cells. As a result, antigen presentation might effectively take place, and the immune response to the virus might be enhanced (the opsonin effect). This possibility is reported by Galili et al. (Nonpatent Reference 3). It has also been reported by Galili et al. with regard to tests on the HIV (Nonpatent Reference 4).

### Patent Reference

[Patent Reference 1] JP2008-537681A

### Nonpatent Reference

[Nonpatent Reference 1] Galili U and Avila JL. α-Gal and Anti-Gal. 1999. Kluwer Academic / Plenum Publishers, New York, N.Y.
[Nonpatent Reference 2] Deriy L, Ogawa H, Gao GP, Galili U. In vivo targeting of vaccinating tumor cells to antigen-presenting cells by a gene therapy method with adenovirus containing the α-1,3galactosyltransferase gene. Cancer Gene Ther. 2005; 12: 528-539.
[Nonpatent Reference 3] Abdel-Motal UM, Guay HM, Wigglesworth K, Welsh RM, Galili U. Immunogenicity of influenza virus vaccine is increased by anti-gal-mediated targeting to antigen-presenting cells. J Virol. 2007; 81: 9131-9141.
[Nonpatent Reference 4] Abdel-Motal U, Wang S, Lu S, Wigglesworth K, Galili U. Increased immunogenicity of human immunodeficiency virus gp 120 engineered to express Ga α1-3Galβ1-4GlcNAc-R epitopes. J Virol. 2006; 80: 6943-6951.
[Nonpatent Reference 5] Eto M, Mase M. Isolation of the Newcastle disease virus and the H9N2 influenza A virus from chicken imported from China. J. Jpn. Vet. Med. Assoc. 2003; 56: 333-339.
[Nonpatent Reference 6] WHO. WHO Manual on Animal Influenza Diagnosis and Surveillance. 2002. WHO, Geneva, Switzerland.
[Nonpatent Reference 7] Killington RA, Stokes A, Hierholzer JC. Virus purification. pp: 71-89. In: Virology Methods Manual. Mahy BWJ, Kangro HO (Ed). 1996. Academic Press Limited, London.

Accordingly, the present invention is characterized by the embodiments as defined in the claims. Thus, it relates to the following items:
1. A method for preparing an α-galactose epitope (Galα1-3Galβ1-4GlcNAc -R: α-Gal hereinafter)-expressing virus, comprising the steps of:
   (1) introducing an α1,3-galactosyltransferase gene in an expressible condition into a cell line that does not express α-Gal to obtain a cell line capable of expressing α-Gal;
   (2) inoculating a virus into the cell line capable of expressing α-Gal to obtain a cell line infected with a virus; and
   (3) culturing the cell line infected with the virus to obtain a virus expressing α-Gal from the culture medium
   wherein the introduction of the α1,3-galactosyltransferase gene is conducted by introducing an expression vector containing an α1,3-galactosyltransferase gene in an expressible condition, and wherein said expression vector has a CAG promoter for expressing the α1,3-galactosyltransferase gene.
2. The method for preparing an α-Gal-expressing virus according to item 1, wherein the cell line that does not express α-Gal is a cell line derived from an organism belonging to the Aves or the Primates.
3. The method for preparing an α-Gal-expressing virus according to any one of items 1 to 2, wherein the α1,3-galactosyltransferase gene is derived from a mouse, pig, or bovine.
4. The method for preparing an α-Gal-expressing virus according to any one of items 1 to 3, wherein the virus is an influenza virus, smallpox virus, measles virus, mumps virus, rubella virus, HIV, Newcastle disease virus, or Marek's disease virus.
5. A method for preparing a vaccine by preparing an α-Gal-expressing virus by the method described in any one of items 1 to 4 and preparing a vaccine from the virus.
6. The method of preparing a vaccine according to item 5, wherein the preparation of the vaccine comprises the step of inactivating the virus.
7. The method of preparing a vaccine according to item 5, wherein the preparation of the vaccine comprises the step of rendering the virus into subunits.
8. The method of preparing a vaccine according to item 5, wherein the preparation of the vaccine comprises the step of attenuating the virus.
9. The method for preparing a vaccine according to any one of items 5 to 8, wherein the preparation of the vaccine comprises the step of incorporating an adjuvant into the virus.

### Problem to be Solved by the Invention

*In vitro* enzymatic reactions have been employed as a method of inducing the expression of α-Gal on viruses (Nonpatent References 3 and 4). However, from perspectives such as that this method requires that an enzyme must be employed in the reaction, that time is required for the enzymatic reaction, that the enzymatic reaction solution must be removed, and the like, the preparation of large quantities of α-Gal-expressing virus for use in vaccines by this method is considered difficult.

Accordingly, one object of the present invention is to provide a method permitting the preparation of an α-Gal-expressing virus that enhances the immune response to the virus. A further object of the present invention is to provide a highly effective (antigenic) vaccine employing the α-Gal-expressing virus prepared by this method, particularly an influenza virus vaccine.

The present inventors discovered that when they introduced the α1,3-galactosyltransferase gene in an expressible condition into a cell line that did not naturally express α-Gal and infected the cell line with a virus, they were able to produce a virus that expressed α-Gal (α-galactose epitope), and that using this virus expressing α-Gal, they obtained a vaccine that elicited an enhanced immune response to the virus relative to a conventional vaccine employing a virus that did not express α-Gal. The present disclosure was devised on that basis.

The present disclosure is as set forth below.
[1] A method for preparing an α-galactose epitope (Galα1-3Galβ1-4GlcNAc -R: α-Gal hereinafter)-expressing virus, comprising the steps of:
   (1) introducing an α1,3-galactosyltransferase gene in an expressible condition into a cell line that does not express α-Gal to obtain a cell line capable of expressing α-Gal;
   (2) inoculating a virus into the cell line capable of expressing α-Gal to obtain a cell line infected with a virus; and
   (3) culturing the cell line infected with the virus to obtain a virus expressing α-Gal from the culture medium.
[2] The method for preparing an α-Gal-expressing virus according to [1], wherein the cell line that does not express α-Gal is a cell line derived from an organism belonging to the Aves or the Primates.
[3] The method for preparing an α-Gal-expressing virus according to [1] or [2], wherein introduction of the α1,3-galactosyltransferase gene is conducted by introducing a vector containing an α1,3-galactosyltransferase gene in an expressible condition.
[4] The method for preparing an α-Gal-expressing virus according to [3], wherein the vector containing an α1,3-galactosyltransferase gene in an expressible state is a expression vector containing the α1,3-galactosyltransferase gene and a CAG promoter or a CMV promoter.
[5] The method for preparing an α-Gal-expressing virus according to any one of [1] to [4], wherein the α1,3-galactosyltransferase gene is derived from a mouse, pig, or bovine.
[6] The method for preparing an α-Gal-expressing virus according to any one of [1] to [5], wherein the virus is an influenza virus, smallpox virus, measles virus, mumps virus, rubella virus, HIV , Newcastle disease virus, or Marek's disease virus.
[7] A method for preparing a vaccine by preparing an α-Gal-expressing virus by the method described in any one of [1] to [6] and preparing a vaccine from the virus.
[8] The method of preparing a vaccine according to [7], wherein the preparation of the vaccine comprises the step of inactivating the virus.
[9] The method of preparing a vaccine according to [7], wherein the preparation of the vaccine comprises the step of rendering the virus into subunits.
[10] The method of preparing a vaccine according to [7], wherein the preparation of the vaccine comprises the step of attenuating the virus.
[11] The method for preparing a vaccine according to any one of [7] to [10], wherein the preparation of the vaccine comprises the step of incorporating an adjuvant into the virus.

No means of obtaining a virus expressing α-Gal by incorporating a gene into a cell that does not originally express α-Gal to induce α-Gal expression and using the cell to grow the virus is currently known to exist.

The present disclosure simply changes a cell that grows a virus from a cell that does not express α-Gal to one that expresses α-Gal, thereby permitting the large-quantity production of a virus expressing α-Gal without the need for a special enzyme or a reaction period.

The fact that α-Gal is expressed on viruses grown in α-Gal-expressing cells is known (Nonpatent Reference 1). However, the present disclosure affords an advantage in that cells are derived from the Aves or the Primates, which used to been employed to obtain vaccine viruses, such as the influenza virus, and do not express α-Gal, are employed to grow many vaccine viruses that can express α-Gal.

The present disclosure provides a virus expressing α-Gal, which is recognized as a foreign antibody by humans and Aves that have natural antibodies against α-Gal.

In addition, by employing the virus expressing α-Gal, the present disclosure provides a vaccine inducing an enhanced immune response to the virus in humans and Aves, which retain natural antibodies to α-Gal, than conventional vaccines obtained using viruses that do not express α-Gal.

### [Brief Description of the Drawings]

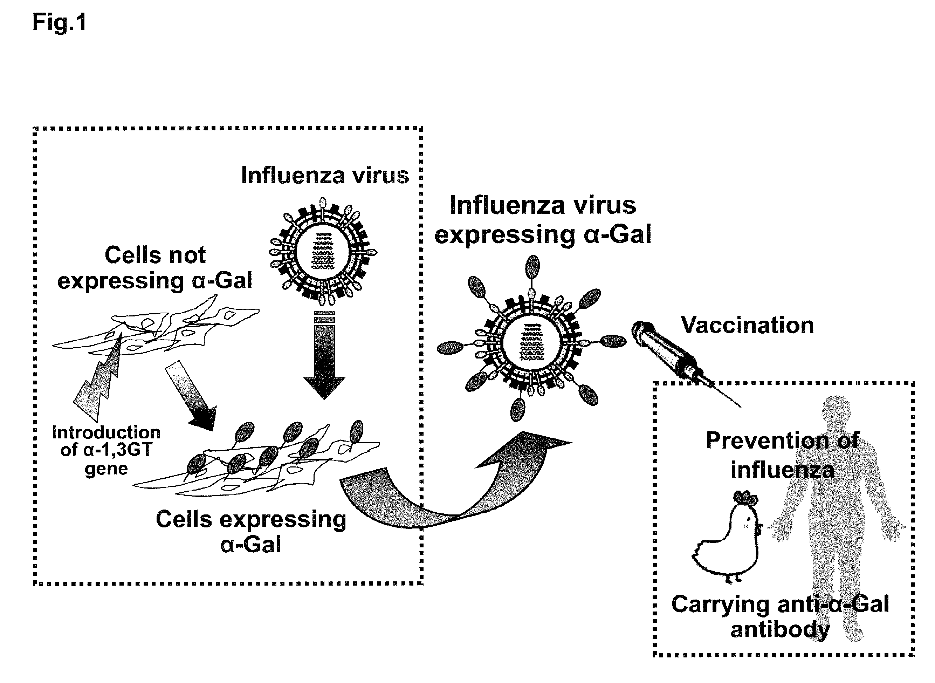

[Figure 1] A schematic drawing that illustrates the method of preparing a virus of the present invention and the use of a vaccine prepared from this virus. This drawing illustrates how an influenza vaccine can be prepared using an α-Gal-expressing cell line and how the obtained vaccine can be used.

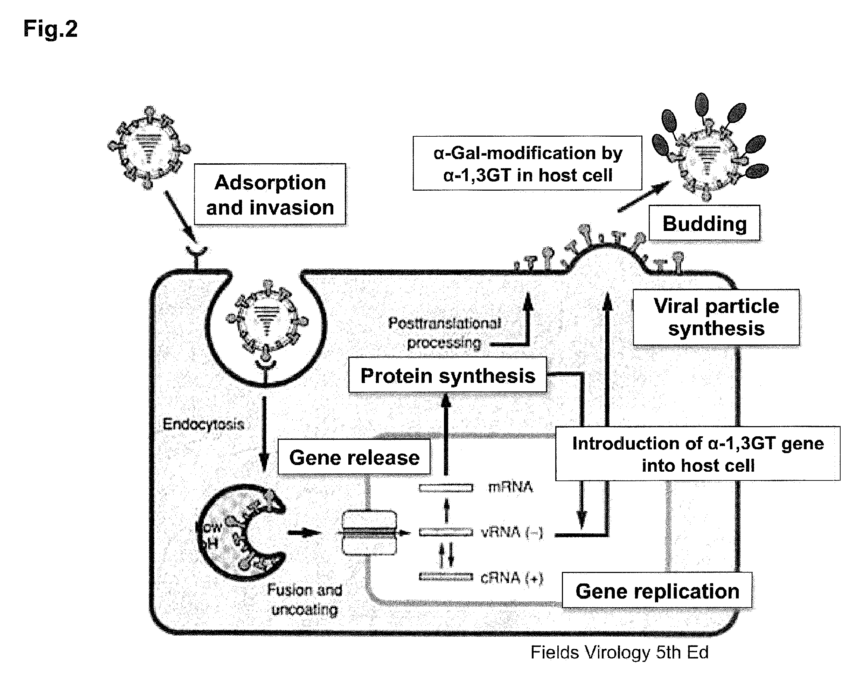

[Figure 2] A schematic drawing that illustrates how an α-Gal-expressing virus can be proliferated by inoculating a virus possessing a glycoprotein on the viral surface into an α-Gal-expressing cell line having α-galactosyltransferase, replicating the influenza virus in cells and adding α-Gal thereto.

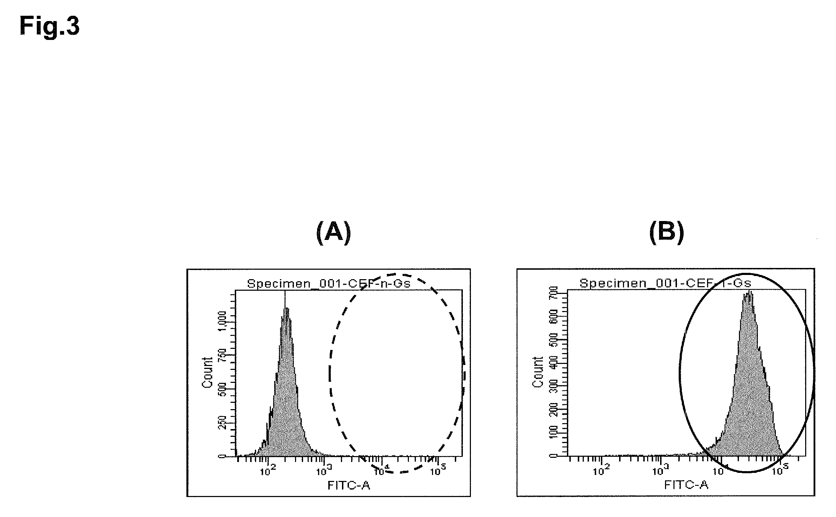

[Figure 3] Shows the results of analysis, by flow cytometry, of α-Gal-expression on the α-Gal-expressing cells of Example 1, i.e., the chicken embryonic fibroblast cell line (CEF-DF1). No cell showed the binding of GS-IB₄ in the original cells (the circle drawn with a broken line) (A). All of the cells into which α1,3G gene had been showed the binding of GS-IB₄ (the circle drawn with a solid line), thus confirming the expression of α-Gal (B).

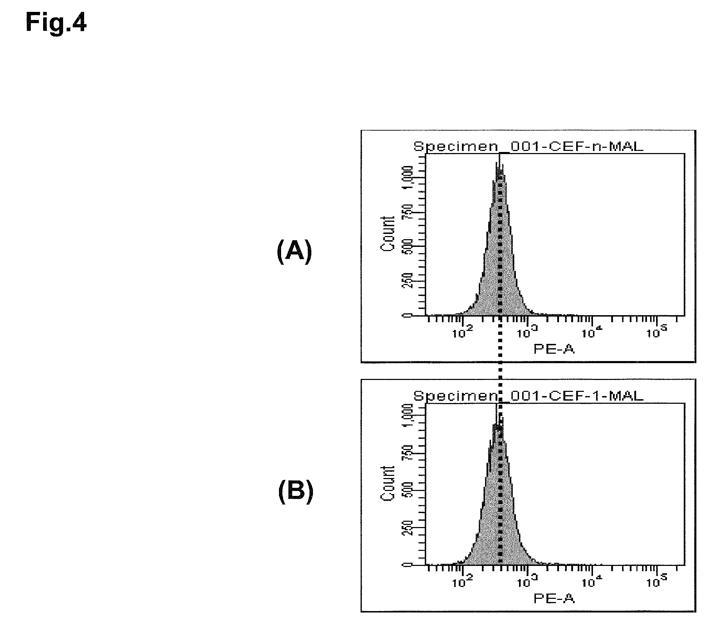

[Figure 4] Shows the results of analysis (the binding of MALII), by flow cytometry, of the sialic acid expression level in the chicken embryonic fibroblast cell line CEF-DF1 in Example 1. No difference was observed in the binding of MALII (α2,3-sialic acid expression) between the original cells (A) and the cells into which α1,3GT gene had been introduced (B).

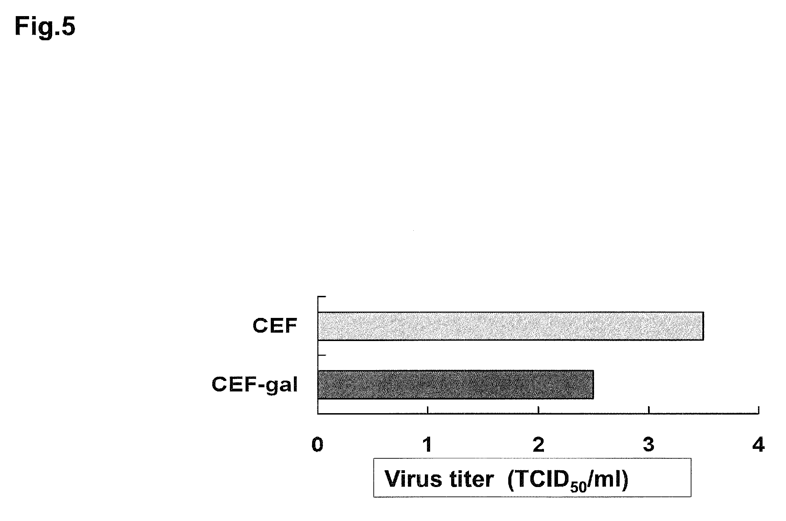

[Figure 5] Shows results relating to the proliferative ability of influenza virus in the α-Gal-expressing cells obtained in Example 1, i.e., the chicken embryonic fibroblast cell line (CEF-DF1). Although the virus having proliferated in the α-Gal-expressing cells (CEF-gal) showed a titer lower by 1-log than the virus having proliferated in the original cells (CEF), the virus titer was maintained at a high level.

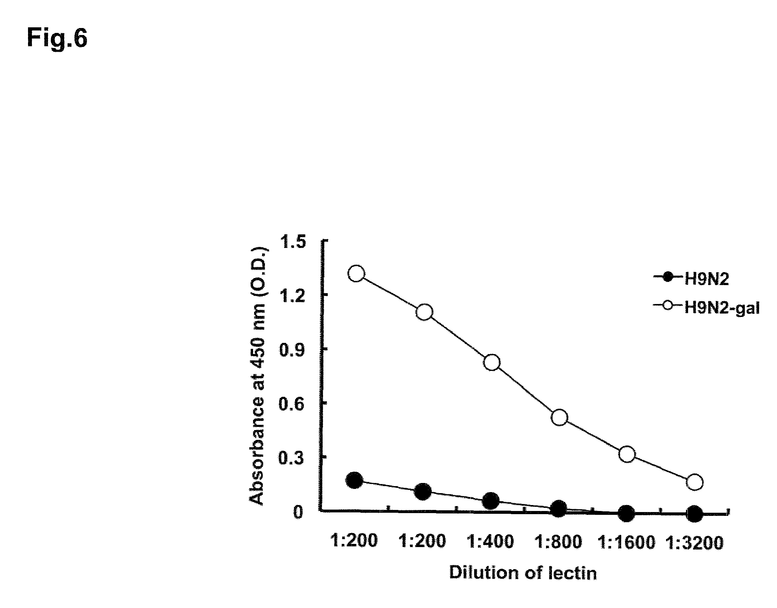

[Figure 6] Shows the results of the confirmation, by analyzing the binding of GS-IB₄ by ELISA, of the acquisition of the H9N2 (H9N2-gal) virus from the α-Gal-expressing chicken embryonic fibroblast cells, in Example 2 as targeted. The H9N2 virus having proliferated in the original cells not expressing α-Gal showed no binding of GS-IB₄. In contrast, the H9N2-gal virus (H9N2-gal) having proliferated in the α-Gal-high-expressing cells obviously showed the binding of GS-IB₄, thus confirming the high expression of α-Gal.

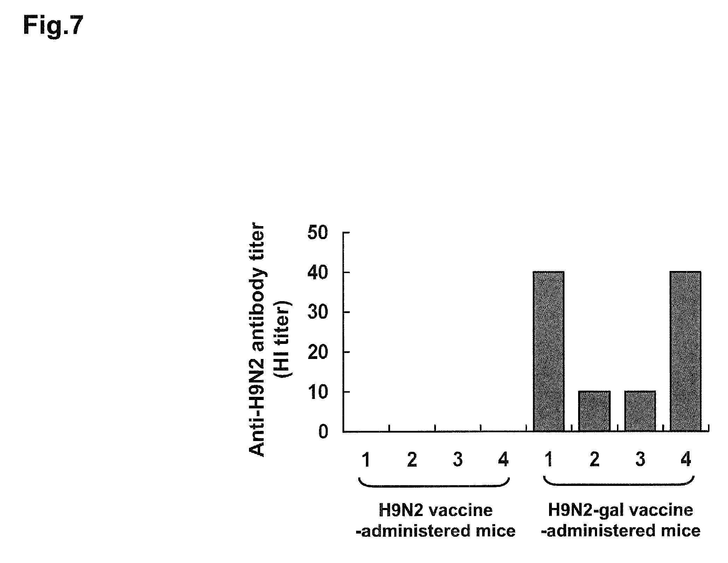

[Figure 7] Shows the results of the confirmation (HI test) of the anti-influenza antibody production in α1,3GT-KO mice (male) to which an α-Gal-expressing influenza vaccine in Example 3 had been administered. These results indicate that no antibody production was confirmed in the mice administered with the H9N2-vaccine, while all of the mice administered with the H9N2-gal vaccine showed the antibody production.

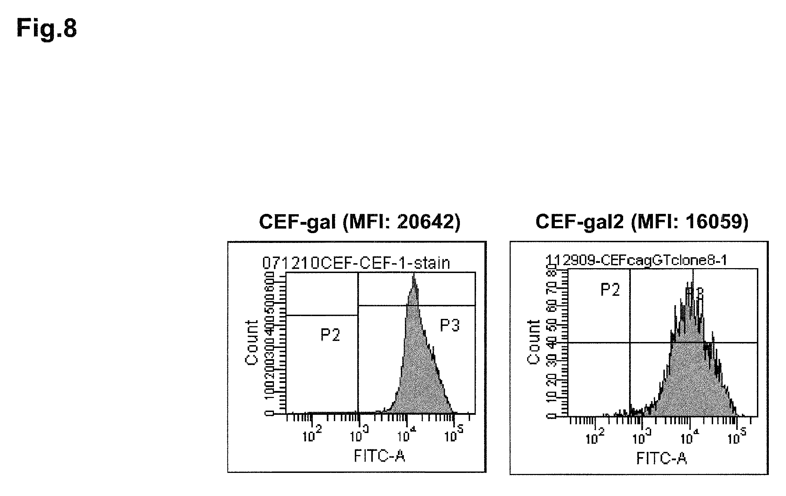

[Figure 8] Shows the results of the analysis (GS-IB₄-staining), by flow cytometry using GS-IB₄, of α-Gal-expression in the cells CEF-gal and CEF-gal2 obtained in Example 5. It was clarified that the α-Gal expression level in CEF-gal2 was somewhat lower than the expression level in CEF-gal. In Figure 8, MFI stands for mean fluorescence intensity.

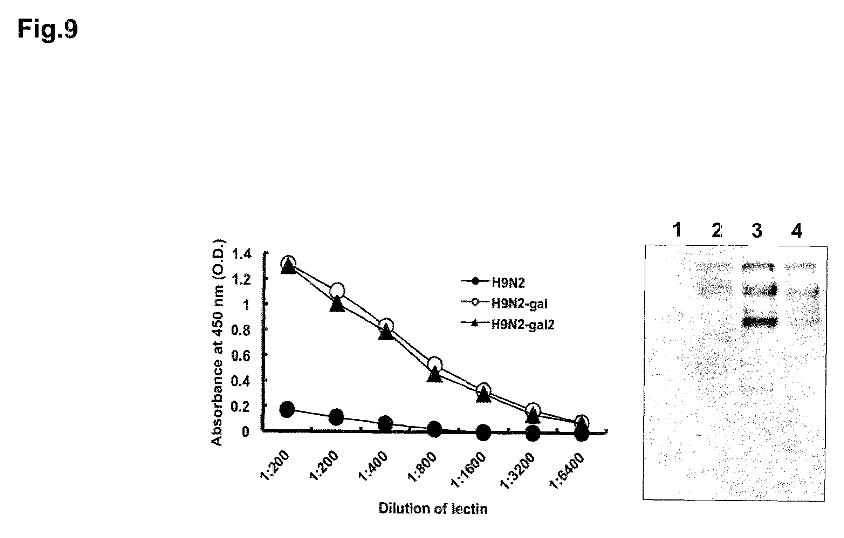

[Figure 9] Shows the results of the confirmation of α-Gal-expression in the H9N2 virus obtained from CEF-gal2 in Example 5, by Western blotting using GS-IB₄ (right) and ELISA (left). The ELISA pattern (left) shows that the α-Gal-expression level in the virus H9N2-gal having proliferated in CEF-gal was almost the same as the α-Gal-expression level in the virus H9N2-gal2 having proliferated in CEF-gal2. The Western blotting pattern (right) also indicates that the α-Gal-expression level in H9N2-gal (lane 2) was almost the same as the α-Gal-expression level in the H9N2-gal2 (lane 4). In contrast, no α-Gal-expression was observed in the H9N2 virus having proliferated in the original cells CEF (left: H9N2, right: lane 1).

The present disclosure primarily relates to a method for preparing a virus expressing α-Gal and to a method for preparing a vaccine using this virus. Figure 1 shows a descriptive schematic drawing for the example of an influenza virus. In the method for preparing a virus expressing α-Gal, the gene coding for α1,3-galactosyltransferase (α 1,3GT), an α-Gal synthetase, is introduced into a cell that does not express α-Gal to change the cell into one that expresses α-Gal. Next, an influenza virus that does not express α-Gal is inoculated and grown, yielding an influenza virus that expresses α-Gal. In the method for preparing a vaccine, this virus is used to prepare a vaccine. The vaccine obtained is employed to prevent influenza in humans and Aves, which have anti-α-Gal antibodies.

### [The method for preparing a virus expressing α-Gal] [0021]

The method for preparing a virus expressing α-Gal of the present disclosure comprises steps (1) to (3) below:
(1) introducing an α1,3-galactosyltransferase gene in an expressible condition into a cell line that does not express α-Gal to obtain a cell line capable of expressing α-Gal;
(2) inoculating a virus into the cell line capable of expressing α-Gal to infect the cell line with the virus; and
(3) culturing the cell line infected with the virus to obtain a virus expressing α-Gal from the culture medium.

α-Galactose epitope (α-Gal) is a sugar chain, denoted by Galα1-3Galβ1-4GlcNAc-R, that is produced by subjecting a glycoprotein (Galβ1-4GlcNAc-R, where R denotes a protein) expressing N-acetyllactosamine to the action of α1,3-galactosyltransferase to generate an α1-3 binding between the galactose substrate and the galactose group of N-acetyllactosamine.

### Step (1)

In step (1), a cell line capable of expressing α-Gal is obtained. The cell line employed in step (1) is one that does not express α-Gal. Examples of cell lines that do not express α-Gal are cell lines derived from Aves and Primates such as humans, apes, and old world monkeys, for example. However, there is no specific limitation, and cell lines from species other than these can be employed in the present disclosure so long as they do not express α-Gal.

The bird from which the cell line is derived is intended to refer to any species, subspecies, or breed of organism of the taxonomical class Aves (including, but not limited to, organisms such as chickens, turkeys, ducks, geese, quail, pheasant, parrots, finches, hawks, crows, ostriches, emus, and cassowaries). This term includes various systems such as the red junglefowl (*Gallus gallus*) and chickens (for example, White Leghorn, Brown Leghorn, Barred-Rock, Sussex, New Hampshire, Rhode Island, Australorp, Minorca, Amrox, California Gray, Italian Partridge-colored), as well as commonly bred turkeys, pheasants, quail, ducks, ostriches, and other domestic fowl systems. The cells derived from Aves that are employed in the preparation method of the present disclosure are desirably chicken cells.

The humans and apes from which cell lines are derived are animals belonging to the superfamily Hominoidea, infraorder Catarrhini, suborder Similiformes of the Primates. Examples in addition to humans are chimpanzees, gorillas, orangutans, and gibbons. This is not intended to be a limitation, however.

The old world monkeys from which cell lines are derived are animals belonging to the super family Cercopithecoidea, infraorder Catarrhini, suborder Similiformes of the Primates. Examples are the Japanese macaque, crab-eating macaque, rhesus macaque, and Hamadryas baboon. This is not intended to be a limitation, however.

The type of cell line is not specifically limited. From the perspective of being used to grow a virus, examples are fibroblast cell lines, epithelial cell lines, and embryonic stem cell lines.

Specific examples of avian-derived cell lines are chicken embryo-derived fibroblast cell lines and chicken embryo-derived stem cell lines. A specific example of an old world monkey-derived cell line is an African green monkey-derived kidney epithelial cell line. A specific example of a primate-derived cell line is a human fetus-derived kidney epithelial cell line. This is not intended to be a limitation, however.

The α1,3-galactosyltransferase gene can be introduced into the α-Gal non-expressing cell line by any known method of introducing a foreign gene, which has been incorporated into an expression vector, into a host cell line. Examples of methods of introducing genes into host cell lines are physicochemical methods such as the calcium phosphate method, lipofection method, electroporation method, and gene gun method, and biological methods employing virus vectors and the like.

Examples of the expression vectors employed in those introduction methods employing expression vectors comprised of: 1) a multicloning site (MCS) for introducing an insertion sequence; 2) a promoter and poly(A) tail sequence for expressing an insertion sequence; 3) drug-resistance marker genes -- promoters and poly(A) tail sequences for expressing them -- for selecting cells into which genes have been introduced; 4) ori sequences that are replicated in prokaryotic cells; 5) antibiotic resistance genes for drug selection in prokaryotic cells; and the like. A variety of such expression vectors are known, and they can be suitably selected for use. Examples of the expression vector that is employed to introduce the gene are pcDNA3.1 comprising a neomycin-resistance gene; pcDNA3.1/Hygro, which is a vector having the same MCS as pcDNA3.1 along with a hygromycin-resistance gene, and pcDNA3.1/Zeo, which has the same MCS along with a zeocin-resistance gene. In addition to the pcDNA series, there are numerous vectors such as the pEF series, pTriEx series, and pLP series. These expression vectors are available as commercial products.

As the expression vector to be employed in the gene introduction, it is preferred to use a vector having a CAG promoter as a promoter for expressing an insertion sequence. A CAG promoter is a hybrid promoter wherein promoter and poly A sequence comprise a cytomegalovirus enhancer, a chicken β-actin promoter, a rabbit β-globin splicing acceptor and a poly A sequence (JP A H03-168087, JP A 2006-121). A CAG promoter, which is one of promoters known as high-expression promoters, may be substituted by other high-expression promoters such as a CMV promoter or an EF1α promoter. Use of an expression vector having the aforesaid high-expression promoter, in particular, CAG promoter enables the acquisition of a cell line with a somewhat suppressed α-Gal-expression level. In the cell line with a somewhat suppressed α-Gal-expression level, influenza virus can proliferate at almost the same level as in the original cells. To the virus having proliferated in the aforesaid cells, moreover, α-Gal can be added in almost the same amount as to the virus having proliferated in α-Gal-high expressing cells.

As Example 1 indicates, in the α-Gal-expressing chicken embryonic embryonic fibroblast cells (CEF-gal), the proliferative ability of influenza virus was somewhat inferior to the proliferative ability in the original cells (CEF) and, therefore, the titer of the virus thus obtained was lower by 1-log (Figure 5). It is presumed that a somewhat decrease in the expression of sialic acid, which serves as an influenza virus receptor, caused by the high-expression of α-Gal might affect in the above phenomenon. In Example 5, in contrast thereto, a promoter inducing the expression of a GT gene was converted into a CAG promoter to give a cell line with a somewhat suppressed α-Gal-expression level. As a result, the titer of the obtained virus was almost the same as the titer of the virus obtained in the original cells (CEF) (Table 1).

There are known α1,3-galactosyltransferase genes derived from mice, swine, cattle, and the like. Gene sequence information is available from GenBank (murine: GenBank accession number M85153; J. Biol. Chem. 267, 5534-5541 (1992) (SEQ. ID NO: 1); porcine: GenBank accession number L36535; Xenotransplantation 1, 81-88, 1994 (SEQ. ID NO: 2); bovine: GenBank accession number J04989; J. Biol. Chem. 264, 14290-14297, 1989 (SEQ. ID NO: 3)).

A vector can be constructed in which the α1,3-galactosyltransferase gene is incorporated as a foreign gene into the MCS of the above expression vector by the usual methods. The constructed vector can be incorporated into an α-Gal non-expressing cell line and screened with an antibiotic to obtain a cell line capable of expressing α-Gal within the cell in which the α1,3-galactosyltransferase gene has been introduced in an expressible condition.

### Step (2)

In step (2), a virus is inoculated into a cell line capable of expressing α-Gal to obtain a virus-infected cell line.

The virus that is employed in the inoculation is not specifically limited so long as it possesses a glycoprotein on the viral surface. The mechanism will be described further below with Figure 2. When the virus having a glycoprotein on the viral surface is inoculated into a cell line having α1,3-galactosyltransferase, it is possible to grow a virus expressing α-Gal.

Examples of viruses having a glycoprotein on the viral surface are: the influenza virus, smallpox virus, measles virus, mumps virus, rubella virus, Newcastle disease virus, and Marek's disease virus. The HIV (a retrovirus) has a glycoprotein on the viral envelope. Although the technique differs from that of the present invention, Galili has reported a method of expressing α-Gal (Nonpatent Reference 4) on HIV. Examples of influenza viruses are the human influenza type A Soviet virus (subtype H1N1), type A Hong Kong virus (subtype H3N2), subtype H5N1 of the highly pathogenic avian influenza virus, and subtype H9N2 of the low pathogenic avian influenza virus. With respect to subtype H1N1 influenza virus, Galili has reported a method for expressing α-Gal, though the method employed therein differs from that of the present invention (Nonpatent Reference 3).

The virus is inoculated into the cell line by the usual methods. For example, an influenza virus can be inoculated into a chicken embryonic fibroblast cell line according to the WHO method (see Nonpatent Reference 7). Specifically, a cell culture medium is employed, the medium of cells that have proliferated in a single layer is removed, and an original solution of virus that does not express α-Gal is inoculated and allowed to adsorb for about 30 minutes. Subsequently, a virus growth medium is added and culturing of the cells is continued to inoculate the virus into the cell line.

### Step (3)

In step (3), the virus-infected cell line obtained in step (2) is cultured to obtain a virus expressing α-Gal from the medium. The term "virus expressing α-Gal" is a virus having α-Gal on the surface thereof. The α-Gal that is present on the surface of the virus is recognized as a foreign antigen by humans and Aves, which have natural antibodies to α-Gal.

A virus expressing α-Gal employing a virus-infected cell line can be prepared as follows, for example. The virus that is inoculated into the cells expressing α-Gal becomes a virus that expresses α-Gal in the course of replicating in cells, and is released to the exterior of the cells. Thus, the virus expressing α-Gal is contained in the cell medium. Several days after inoculating the cells with the virus, cytopathic effects (CPEs) caused by the virus can be observed by microscopy. The cells are cultured to a stage where CPEs are adequately observable. Subsequently, a cell culture supernatant containing an abundance of α-Gal-expressing virus is recovered.

The fact that a virus expressing α-Gal and having α-Gal on the surface thereof can be prepared by infecting a cell line that is capable of expressing α-Gal with a virus will be specifically described using Figure 2. Generally, the constituent proteins of virus particles are synthesized within cells that have been inoculated with a virus (upper left in figure). However, when these are "glycoproteins," the "sugars" that are synthesized by glycotransferase that is present within the cell are added to proteins. Thus, α-Gal is added to a glycoprotein that is synthesized in cells in which α-galactosyltransferase is functional. Accordingly, when a virus having a glycoprotein on the surface thereof (such as an influenza virus, measles virus, or HIV) is inoculated into α-Gal-expressing cells (having α-galactosyltransferase), it is possible to grow a virus that expresses α-Gal.

The α-Gal-expressing virus that is obtained by the preparation method of the present invention can induce an enhanced immune response to the virus in humans and Aves, which have natural antibodies to α-Gal, relative to viruses of the same type which do not express α-Gal. They exhibit what are known as opsonin effects. In the case of an α-Gal-expressing virus, this is thought to occur because anti-Gal antibodies bind to the virus in humans and Aves, and then bind to the Fcγ receptors of antigen-presenting cells. As a result, effective antigen presentation takes place.

### [The method for preparing a vaccine]

The present invention covers a method for preparing a vaccine using an α-Gal-expressing virus obtained by the preparation method of the present invention set forth above.

As stated above, an α-Gal-expressing virus is recovered as a cell culture supernatant containing an abundance of α-Gal-expressing virus. The cell culture supernatant can be employed as is to prepare a vaccine, or can be purified by the usual methods. One example of a purification method is ultracentrifugation using a sucrose solution by the method described in Nonpatent Reference 7.

The virus obtained by the preparation method of the present invention can be an inactivated virus, for example. The virus contained in the vaccine can also consist of viral subunits. The virus contained in the vaccine can also be an attenuated virus.

The virus can be suitably inactivated by a known virus inactivation processing method. Examples of inactivation processing are subjecting a purified virus to formalin, UV radiation, or β-propiolactone to inactivate it.

Viral subunits (components) can be suitably prepared by a known method. For example, a viral solution is mixed with 1% Tween 20 in a 9:1 ratio. The mixture is left standing for 30 minutes, after which an equal quantity of ether is added and the mixture is vigorously stirred. The mixture is then centrifuged to obtain an aqueous phase fraction, which is inactivated by the same method as that set forth above to obtain a subunit vaccine.

An attenuated virus can be obtained by reducing its pathogenicity by a known method such as genetic mutation. However, for viruses that are prone to mutation, such as the influenza virus, the use of an attenuated virus presents numerous concerns. Thus, vaccines of inactivated virus and subunit vaccines are generally employed instead of vaccines of attenuated virus.

The vaccine that is obtained by the preparation method of the present invention will sometimes contain just the above virus, and sometimes further contain an adjuvant. Examples of adjuvants are vegetable oils such as sesame oil and rapeseed oil; mineral oils such as light liquid paraffin; aluminum hydroxide gel; and aluminum phosphate gel.

Examples of the route of administration of the vaccine obtained by the preparation method of the present invention are eyedrops, nosedrops, intramuscular, and subcutaneous. When administered as an inactive vaccine, intramuscular, intra-abdominal, and subcutaneous administration are desirable.

The vaccine obtained by the preparation method of the present invention can be employed to prevent and/or treat humans and Aves infected with the virus.

### Examples

The present invention will be described in greater detail below through Examples.

### Test methods

Next, the test methods employed in the following Examples will be described.

### (1a) Flow cytometry using Griffonia Simplicifolia lectin I-Isolectin B₄ (GS-IB₄)

α-Gal expressed on cells was stained with fluorescein isothiocyanate (FITC)-labeled GS-IB₄ (Vector Laboratories Inc.) for 30 min at 4°C, washed and then analyzed by FACS Canto flow cytometer (BD Biosciences).

### (1b) Flow cytometry using Maackia Amurensis lectin II (MALII) and Sambucus Nigra lectin (SNA)

α2,3-Sialic acid or α2,6-sialic acid was reacted respectively with biotinylated MALII (Vector) or biotinylated SNA (Vector) for 30 min at 4°C, washed and then stained with Phycoerythrin (PE)-labeled streptavidin (Vector). The stained cells were analyzed by FACS Canto flow cytometer (BD Biosciences).

### (2) ELISA method

ELISA method for examining α-Gal expressed on viruses was conducted as follows. A purified virus was mixed with a solution containing 2% Triton X-100 and 1 M KCl at a ratio of 9:1 and the resultant mixture was allowed to stand for 30 min at 4°C. The obtained solution was diluted with carbonate buffer (pH 9.6) to give a 5 µg/ml virus solution. Then, a 96-well ELISA plate was coated with the solution at 50 µl/well. After blocking with 1% bovine serum albumin-containing PBS (BSA/PBS), the virus solution was reacted with HRP-labeled GS-IB₄ (Sigma-Aldrich) for 1 hour at room temperature. After color-developing with tetramethyl benzidine (TMB: BD Bioscience), the absorbance (O.D. 450 nm) was measured.

### (3) Western blotting method

Western blotting method for examining α-Gal expressed on viruses was conducted as follows. Viral proteins were separated by sodium dodecylsulfate-polyacrylamide gel electrophoresis (SDS-PAGE) in the presence of 2-mercaptoethanol, then transferred on a polyvinylidene difluoride (PVDF) membrane (Bio-Rad) and blocked with 1% alkali-soluble casein (Novagen). After reacting with HRP-labeled GS-IB₄ (Sigma-Aldrich) for 1 hour at room temperature, the PVDF membrane thus reacted was visualized by ECL Western blotting analysis system (Amersham Biosciences) and analyzed with the use of LAS-3000 (Fuji Film).

### (4) HI test method

Anti-H9N2 antibody titer in mouse plasma was evaluated by HI test. Detailed procedures of the test are as follows.

Mouse serum was treated with RDE (Denka Seiken) to remove non-specific hemagglutination inhibitors. Next, chicken erythrocytes were mixed with the plasma at a ratio by volume of 1:5 to remove spontaneous agglutinins from the plasma. After centrifuging, the obtained supernatant was subjected, as a treated plasma sample, to the HI test. In the test, the plasma sample was serially diluted (1:2) in a V-bottomed 96-well microplate and a virus solution containing 4 U of hemagglutinin (HA) was added, in the same amount (25 µl) as the plasma sample, to each well. After reacting for 1 hour at room temperature, a 0.5% chicken erythrocyte solution (50 µl) was added and the hemagglutination was observed.

### Example 1

### Cell line in which α-Gal expression is induced by introducing a virus

Using pcDNA3.1(+) (Invitrogen) into which a murine α1,3-galactosyltransferase (α1,3GT) gene had been incorporated, an α1,3GT gene was introduced into a chicken embryonic fibroblast cell line CEF-DF1 (ATCC CRL-12203: "CEF" herein after). Dulbecco modified Eagle's medium to which bovine fetal serum, L-glutamine, penicillin, and streptomycin had been added was used to culture the CEF. The day after gene introduction, the cells were subcultured. Selectioning was conducted with 100 µg/mL of G418 from the next day on. Cloning was conducted after two weeks. Cell culturing following sectioning was conducted with G418 added to the medium.

The expression of α-Gal in the cell clones obtained was evaluated by analyzing the binding of *Griffonia Simplicifolia* lectin I-Isolectin B₄ (GS-IB₄, Vector Laboratories, Inc.) by flow cytometry, ELISA, or Western blotting. Figure 3 shows the results of flow cytometry.

The expression of sialic acid was evaluated by analyzing the binding of *Maackia Amurensis* lectin II (MALII, Vector) and *Sambucus Nigra* lectin (SNA, Vector) by flow cytometry.

No change in the expression level of sialic acid was found in analysis by flow cytometry. The results are shown in Figure 4.

Since both α-Gal and sialic acid are terminal structures of sugar chains and utilize the same substrate, it was predicted that α-Gal expression could be used to reduce the expression of sialic acid. The sialic acid that is expressed by cells serves a receptor in the course of infection by the influenza virus. Thus, sialic acid expression is necessary in cells in which influenza virus is to be grown. That is, a major drop in sialic acid expression due to the expression of α-Gal was undesirable. In the flow cytomteric analysis conducted in the present invention, no decrease in sialic acid expression caused by the expression of α-Gal was observed. However, the fact that the proliferative ability of the influenza virus in the α-Gal-expressing chicken embryonic fibroblast cells (CEF-gal) was somewhat inferior to the proliferative ability in the original cells (CEF) suggests sialic acid expression might be somewhat lowered by the high-expression of α-Gal. Therefore, it is required to establish a cell line enabling the sufficient proliferation of influenza virus. Although the α-Gal-expressing cells obtained were somewhat inferior to the original cells in this regard, they were able to adequately grow the influenza virus. The results are given in Figure 5.

Cell clones that expressed α-Gal and proliferated well were employed as the α-Gal-expressing CEF cell line (CEF-gal).

### Example 2

### Virus expressing α-Gal growing in cell line made to express α-Gal

The CEF-gal prepared in Example 1 was infected with subtype H9N2 avian influenza virus (A/chicken/Yokohama/aq55/01:H9N2 virus) (see Nonpatent Reference 5). Several days later, the culture supernatants of cells exhibiting cytopathic effects were collected as a virus solution. The mediun of cells inoculated with the virus were replaced with virus growth medium in accordance with the WHO method (Nonpatent Reference 6).

The acquisition of the targeted α-Gal-expressing H9N2 virus was confirmed by analyzing the binding of GS-IB₄ by flow cytometry, ELISA or Western blotting. Fig. 6 shows the results of the confirmation, by analyzing the binding of GS-IB₄ by ELISA, of α-Gal expression on the influenza virus obtained from the α-Gal-expressing chicken embryonic fibroblast cells. The H9N2 virus having proliferated in the original cells not expressing α-Gal showed no binding of GS-IB₄. In contrast, the H9N2-gal virus having proliferated in the α-Gal-expressing cells obviously showed the binding of GS-IB₄, indicating the high expression of α-Gal.

Example 2 gives the example of the H9N2 virus. However, a virus expressing α-Gal can be obtained by the same method with other influenza viruses.

### Example 3

### Preparation of α-Gal-expressing virus

The virus solution prepared in Example 2 above was purified by ultracentrifugation using a sucrose solution according to the usual method (Nonpatent Reference 8). The purified virus obtained was inactivated with formalin, UV radiation, or β-propiolactone and an inactivated vaccine was prepared.

As a subunit vaccine, the virus solution was mixed with 1% Tween 20 in a ratio of 9:1. The mixture was left standing for 30 minutes, after which an equal quantity of ether was added. The mixture was vigorously stirred and centrifuged to obtain an aqueous phase fraction, which was inactivated by the same method as that set forth above for use. These vaccines were employed independently, or mixed with adjuvants (Ribi adjuvant system, Corixa: Abisco, Isconova) to obtain vaccines for use.

### Example 4

### Evaluation of α-Gal-expressing virus vaccines

α1,3GT gene knockout mice (α1,3GT-KO mice) retaining anti-α-Gal antibodies and not expressing α-Gal in the same manner as humans and Aves were employed in the test. A 1.0 µg quantity of each of a vaccine of H9N2 that did not express α-Gal and a vaccine of H9N2-gal that did express α-Gal were admixed to an Ribi adjuvant system and administered subcutaneously to the mice. The vaccines employed were subunit vaccines that had been inactivated with formalin. The vaccines were administered twice at an interval of two weeks. Subsequently, the anti-H9N2 antibody titer in mouse plasma was evaluated by a HI test. As a result, no antibody production was confirmed in the mice administered the H9N2 vaccine, but antibody production was confirmed in all of the mice administered the H9N2-gal vaccine. The results are given in Figure 7.

### Example 5

An expression vector, pCAGGS carrying mouse α1,3 GT gene incorporated therein was transfected, together with pcDNA3.1(+), into CEF-DF1 using FuGENE HD and a cell clone (CEF-gal2) having the α1,3 GT gene incorporated into the chromosome was selected by the same method as in Example 1. The α-Gal expression level in the obtained cells was confirmed by flow cytometry using GS-IB₄. Figure 8 shows the results. CEF-gal2 was infected with the H9N2 virus and the titer of the thus obtained virus solution was measured. Thus, the proliferative ability of the virus in the cells was evaluated. Table 1 shows the results. Further, the expression of α-Gal in the H9N2 virus obtained from CEF-gal2 was confirmed by ELISA using GS-IB₄ and Western blotting. Figure 9 shows the results.

**[Table 1]**

| Cells | TCID₅₀/ml |
|---|---|
| (Experiment 1) | |
| CEF | 3.5 |
| CEF-gal | 2.5 |
| (Experiment 2) | |
| CEF | 4.25 |
| CEF-gal2 | 4.25 |

Figure 8 shows the results of the analysis (GS-IB₄-staining), by flow cytometry, of α-Gal-expression in the cells CEF-gal and CEF-gal2. It was clarified that the expression level of α-Gal on the cell surface of CEF-gal2 was somewhat lower than in CEF-gal. In Figure 8, MFI stands for mean fluorescence intensity.

Figure 9 shows the results of the analysis of α-Gal-expression (the binding of GS-IB₄) on the H9N2 virus having proliferated in various cells, by ELISA and Western blotting. The ELISA pattern (left) shows that the α-Gal-expression level on the virus H9N2-gal having proliferated in CEF-gal was almost the same as the α-Gal-expression level on the virus H9N2-gal2 having proliferated in CEF-gal2. The Western blotting pattern (right) also indicates that the α-Gal-expression level on H9N2-gal (lane 2) was almost the same as the α-Gal-expression level on H9N2-gal2 (lane 4). In contrast, no α-Gal-expression was observed on the H9N2 virus having proliferated in the original cells CEF (left: H9N2, right: lane 1).

Moreover, Table 1 shows the titer of virus having proliferated in CEF-gal2. The titer of the virus having proliferated in CEF-gal2 was almost the same as the titer of the virus having proliferated in the original cells (CEF) (Experiment 2), confirming that the virus proliferated at almost the same level. In contrast, the titer of the virus having proliferated in CEF-gal was lower by 1-log (Experiment 1). Thus, it can be confirmed that the H9N2 virus can proliferate in CEF-gal2 at almost the same level in the original cells (CEF) (Table 1). In Table 1, TCID₅₀ stands for the tissue culture infectious dose (virus titer).

As discussed above, it can be confirmed that using α-Gal-expressing CEF-gal2, the targeted influenza virus highly expressing α-Gal can be obtained in almost the same amount as in the original cells. This result suggests that influenza vaccines could be possibly manufactured on a mass scale using α-Gal-expressing cells.

### Industrial Applicability

The present invention is useful in the field of manufacturing vaccines from influenza virus and the like.

## Claims

1. A method for preparing an α-galactose epitope ( α-Gal hereinafter)-expressing virus, comprising the steps of:
(1) introducing an α1,3-galactosyltransferase gene in an expressible condition into a cell line that does not express α-Gal to obtain a cell line capable of expressing α-Gal;
(2) inoculating a virus into the cell line capable of expressing α-Gal to obtain a cell line infected with a virus; and
(3) culturing the cell line infected with the virus to obtain a virus expressing α-Gal from the culture medium
wherein the introduction of the α1,3-galactosyltransferase gene is conducted by introducing an expression vector containing an α1,3-galactosyltransferase gene in an expressible condition, and wherein said expression vector has a CAG promoter for expressing the α1,3-galactosyltransferase gene.

2. The method for preparing an α-Gal-expressing virus according to claim 1, wherein the cell line that does not express α-Gal is a cell line derived from an organism belonging to the Aves or the Primates.

3. The method for preparing an α-Gal-expressing virus according to any one of claims 1 to 2, wherein the α1,3-galactosyltransferase gene is derived from a mouse, pig, or bovine.

4. The method for preparing an α-Gal-expressing virus according to any one of claims 1 to 3, wherein the virus is an influenza virus, smallpox virus, measles virus, mumps virus, rubella virus, HIV, Newcastle disease virus, or Marek's disease virus.

5. A method for preparing a vaccine by preparing an α-Gal-expressing virus by the method described in any one of claims 1 to 4 and preparing a vaccine from the virus.

6. The method of preparing a vaccine according to claim 5, wherein the preparation of the vaccine comprises the step of inactivating the virus.

7. The method of preparing a vaccine according to claim 5, wherein the preparation of the vaccine comprises the step of rendering the virus into subunits.

8. The method of preparing a vaccine according to claim 5, wherein the preparation of the vaccine comprises the step of attenuating the virus.

9. The method for preparing a vaccine according to any one of claims 5 to 8, wherein the preparation of the vaccine comprises the step of incorporating an adjuvant into the virus.

## Patentansprüche

1. Verfahren zur Herstellung eines Virus, das ein α-Galactose-Epitop (im Folgenden: α-Gal) exprimiert, umfassend die Schritte des:
(1) Einführens eines α-1,3-Galactosyltransferase-Gens in exprimierbarem Zustand in eine Zelllinie, die α-Gal nicht exprimiert, um eine Zelllinie zu erhalten, die fähig ist, α-Gal zu exprimieren;
(2) Inokulierens eines Virus in die Zelllinie, die fähig ist, α-Gal zu exprimieren, um eine mit einem Virus infizierte Zelllinie zu erhalten; und
(3) Züchtens der mit dem Virus infizierten Zelllinie, um aus dem Kulturmedium ein Virus zu erhalten, das das α-Gal exprimiert;
wobei das Einführen des α-1,3-Galactosyltransferase-Gens durchgeführt wird durch das Einführen eines Expressionsvektors, der ein α-1,3-Galactosyltransferase-Gen in exprimierbarem Zustand enthält, und wobei der Expressionsvektor einen CAG-Promotor für das Exprimieren des α-1,3-Galactosyltransferase-Gens hat.

2. Verfahren zur Herstellung eines α-Gal exprimierenden Virus nach Anspruch 1, wobei die Zelllinie, die α-Gal nicht exprimiert, eine Zelllinie ist, die von einem Organismus stammt, der zu den Vögeln (Aves) oder den Primaten gehört.

3. Verfahren zur Herstellung eines α-Gal exprimierenden Virus nach einem der Ansprüche 1 bis 2, wobei das α-1,3-Galactosyltransferase-Gen von einer Maus, einem Schwein oder einem Rind stammt.

4. Verfahren zur Herstellung eines α-Gal exprimierenden Virus nach einem der Ansprüche 1 bis 3, wobei das Virus ein Influenza-Virus, Pockenvirus, Masernvirus, Mumpsvirus, Rötelnvirus, HIV, Virus der Newcastle-Krankheit oder Marek-Krankheitsvirus ist.

5. Verfahren zur Herstellung eines Impfstoffs durch Herstellung eines α-Gal exprimierenden Virus durch das in einem der Ansprüche 1 bis 4 beschriebene Verfahren und Herstellung eines Impfstoffes aus dem Virus.

6. Verfahren zur Herstellung eines Impfstoffes nach Anspruch 5, wobei die Herstellung des Impfstoffes den Schritt der Inaktivierung des Virus umfasst.

7. Verfahren zur Herstellung eines Impfstoffes nach Anspruch 5, wobei die Herstellung des Impfstoffes den Schritt der Herstellung von Virus-Untereinheiten umfasst.

8. Verfahren zur Herstellung eines Impfstoffes nach Anspruch 5, wobei die Herstellung des Impfstoffes den Schritt der Attenuierung des Virus umfasst.

9. Verfahren zur Herstellung eines Impfstoffes nach einem der Ansprüche 5 bis 8, wobei die Herstellung des Impfstoffes den Schritt des Inkorporierens eines Hilfsstoffes in den Virus umfasst.

## Revendications

1. Procédé pour préparer un virus exprimant un épitope α-galactose (ci-après α-Gal), comprenant les étapes de :
(1) introduire un gène de l'α1,3-galactosyltransférase, dans une condition où il peut être exprimé, dans une lignée cellulaire qui n'exprime pas l'α-Gal afin d'obtenir une lignée cellulaire capable d'exprimer l'α-Gal ;
(2) inoculer un virus dans la lignée cellulaire capable d'exprimer l'α-Gal afin d'obtenir une lignée cellulaire infectée par un virus ; et
(3) mettre en culture la lignée cellulaire infectée par le virus afin d'obtenir un virus exprimant l'α-Gal à partir du milieu de culture
dans lequel l'introduction du gène de l'α1,3-galactosyltransférase est réalisée en introduisant un vecteur d'expression contenant un gène de l'α1,3-galactosyltransférase dans une condition où il peut être exprimé, et dans lequel ledit vecteur d'expression possède un promoteur CAG pour exprimer le gène de l'α1,3-galactosyltransférase.

2. Procédé pour préparer un virus exprimant l'α-Gal selon la revendication 1, dans lequel la lignée cellulaire qui n'exprime pas l'α-Gal est une lignée cellulaire dérivée d'un organisme appartenant à la classe des Aves ou à l'ordre des Primates.

3. Procédé pour préparer un virus exprimant l'α-Gal selon l'une quelconque des revendications 1 à 2, dans lequel le gène de l'α1,3-galactosyltransférase est dérivé d'une souris, d'un porc ou d'un bovin.

4. Procédé pour préparer un virus exprimant l'α-Gal selon l'une quelconque des revendications 1 à 3, dans lequel le virus est un virus de la grippe, un virus de la variole, le virus de la rougeole, le virus des oreillons, le virus de la rubéole, le VIH, le virus de la maladie de Newcastle, ou le virus de la maladie de Marek.

5. Procédé pour préparer un vaccin en préparant un virus exprimant l'α-Gal par le procédé décrit dans l'une quelconque des revendications 1 à 4 et en préparant un vaccin à partir du virus.

6. Procédé pour préparer un vaccin selon la revendication 5, dans lequel la préparation du vaccin comprend l'étape qui consiste à inactiver le virus.

7. Procédé pour préparer un vaccin selon la revendication 5, dans lequel la préparation du vaccin comprend l'étape qui consiste à dissocier le virus en sous-unités.

8. Procédé pour préparer un vaccin selon la revendication 5, dans lequel la préparation du vaccin comprend l'étape qui consiste à atténuer le virus.

9. Procédé pour préparer un vaccin selon l'une quelconque des revendications 5 à 8, dans lequel la préparation du vaccin comprend l'étape qui consiste à incorporer un adjuvant dans le virus.
